# EUROPEAN PATENT SPECIFICATION

(11) **EP 0 764 023 B1**
(45) Date of publication and mention of the grant of the patent: **07.08.2002**
(21) Application number: 95920874.5
(22) Date of filing: 24.05.1995
(51) Int. Cl.: A61K 31/35

(54) **COMPOUNDS AND COMPOSITIONS FOR ADMINISTRATION VIA ORAL INHALATION OR INSUFFLATION**
VERBINDUNNGEN UND ZUSAMMENSETZUNGEN ZUR VERABREICHUNG VIA INHALATION UND INSUFFLIERUNG
COMPOSES ET COMPOSITIONS DESTINES A ETRE ADMINISTRES PAR INSUFFLATION OU INHALATION ORALE

(30) Priority: 28.05.1994 GB 9410817
(43) Date of publication of application: 26.03.1997
(73) Proprietor: BIOTA SCIENTIFIC MANAGEMENT PTY. LTD., Melbourne, VIC 3004 (AU)
(72) Inventor: EFTHYMIOPOULOS, Constantin, Greenford, Middlesex UB6 0HE (GB)
(74) Representative: Woodman, Derek
(86) International application number: EP9501967
(87) International publication number: WO9532712

(56) References cited:
- EP-A- 0 341 735
- EP-A- 0 539 204
- WO-A-91/16320
- HAYDEN ET AL : "Efficacy and safety of the neuraminidase inhibitor Zanamivir", NEW ENG.J.MED, , 1997, vol. 337, no. 13, pages 874 to -880

## Description

The present invention relates to administration of medicaments by mouth via inhalation or insufflation.

PCT/AU91/00161 (publication no. WO91/16320) describes a number of derivatives of 5-acetamidino-2,3,5-trideoxy-D-glycero-D-galacto-non-2-enopyranosonic acid (2,3,-dideoxy-2,3-didehydro-N-acetyl-neuraminic acid; DANA) including the 4-guanidino analogue of DANA, which has the following structure: 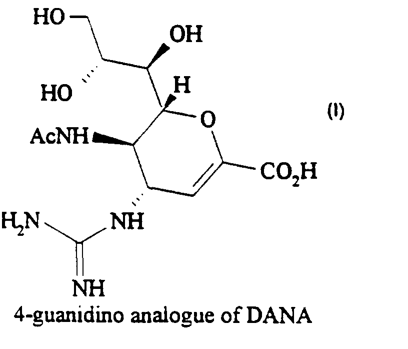 and the chemical name 5-acetamido-2,3,4,5-tetradeoxy-4-guanidino-D-glycero-D-galacto-non-2-enopyranosonic acid, and is also known as GG167.

The compound of formula (I) has antiviral activity. In particular, this compound is an inhibitor of viral neuraminidase, for example, the viral neuraminidase of influenza A and B.

Pharmaceutical formulations, in particular formulations for intranasal administration, are also described in WO91/16320.

We have now found that the compound of formula (I) exhibits antiviral activity in animals, in particular in humans, when administered by mouth via inhalation or insufflation.

The invention provides the use of the compound of formula (I) for the manufacture of a medicament adapted for inhalation or insufflation through the mouth for the treatment of a viral infection.

The compound of formula (I) may be administered by any of the methods and formulations employed in the art for administration by inhalation or insufflation through the mouth.

Thus in general the compound of formula (I) may be administered to the lung in the form of a solution or a suspension or a dry powder. The compound of formula (I) may be micronised or non-micronised. The delivery systems currently available e.g. pressurised metered dose inhalers, nebulisers and dry powder inhalers, are suitable for administration according to the invention. It is anticipated that any system developed in the future for the delivery of dry powder, solution or suspension by inhalation via the mouth will also be suitable.

When desired the formulations may be adapted to give sustained release of the active ingredient.

Solutions and suspensions may be aqueous, for example prepared from water alone (for example sterile or pyrogen - free water), or water and a physiologically acceptable co-solvent (e.g. ethanol, propylene glycol or a polyethylene glycol such as PEG 400). Alternatively, solutions and suspensions may be nonaqueous, for example prepared from organic solvents such as chlorofluorocarbons and fluorocarbons, for example 1,1,1,2 - tetrafluoroethane.

Such solutions and suspensions may additionally contain other excipients for example preservatives (such as benzalkonium chloride), solubilising agents/surfactants such as polysorbates (e.g. Tween 80, Span 80 or lecithins), buffering agents, isotonicity - adjusting agents (e.g. sodium chloride or sugars) and absorption enhancers. Suspensions may additionally contain suspending agents (e.g. microcrystalline cellulose, carboxymethyl cellulose sodium).

Solutions may be preserved or may be aseptically prepared or sterilised after manufacture using conventional methods.

The compound of formula (I) is preferably administered by means of a dry powder inhaler. This method of administration provides particularly rapid delivery of GG167 to the lung.

When the compound of formula (I) is provided in the form of a dry powder, it may be presented alone or in admixture with a suitable pharmaceutically acceptable diluent such as starch, starch derivatives such as hydroxypropylmethyl cellulose or polyvinylpyrrolidine (PVP), sugar derivatives such as mannitol or, preferably, lactose. The powder composition may be presented in unit dose form, for example in capsules or cartridges of e.g. gelatin or. formed plastic or blister packs from which the powder may be administered by means of an inhalation device, or in multidose form from, for example, a powder reservoir.

Suitable inhalation devices include those described in EP 069715, GB 2041763, WO91/13646, GB 1561835, GB 2064336, GB 2129691, GB 2178965 or GB 2242134. A preferred inhalation device for administration in accordance with the invention is the DISKHALER (trade mark). Devices may deliver single or multiple doses.

Dry powder inhalers are designed to deliver a fixed unit dosage of medicament per actuation. When the compound of formula (I) is administered by means of a dry powder inhaler it will suitably be administered in an amount of 0.01 to 25mg, such as 0.5 to 20mg per actuation, for example 0.1 to 10mg per actuation, preferably about 5mg or about 10mg per actuation.

It will be appreciated that the precise dose administered will depend on the age and condition of the patient and the frequency of administration and will ultimately be at the discretion of the attendant physician. Typically, administration may be one or more times, for example 1 to 8 times per day, giving for example 1, 2, 3 or 4 unit doses each time.

We have found that it is possible to administer the compound of formula (I) to a patient using a combination of intranasal administration and inhalation or insufflation via the mouth. For use in combination with inhalation or insufflation via the mouth, intranasal administration may be effected using any of the methods known in the art for intranasal administration of pharmaceuticals and, in particular, any of the methods described in WO91/16320. Thus, for example, the compound of formula (I) may be applied to nasal cavity as a solution, a suspension or a dry powder. Solutions and suspensions may be administered intranasally using, for example, a pipette, a dropper or a spray. Dry powders may be administered intranasally by inhalation, for example, using an inhaler.

A method of combined administration comprises inhalation or insufflation by mouth of GG167 in the form of a dry powder and administration of a solution or suspension of GG167 to the nasal cavity as a spray.

For combined administration, GG167 may be administered by inhalation or insufflation via the mouth and by intranasal administration either simultaneously (i.e. within 10 minutes of each other, such as within about 5 minutes of each other) or separately. Typically GG167 may be administered by inhalation or insufflation via the mouth from 1 to 8 times daily and by intranasal administration from 1 to 8 times daily. Preferably administration via the mouth and intranasal administration will take place essentially simultaneously.

The invention is further illustrated by the following examples.

### Example 1

A double-blind, randomised, placebo-controlled study was conducted in adult human patients all of whom had had symptoms of influenza-like illness (including feverishness and at least two of myalgia, headache, cough, sore throat) for up to 48 hours.

Patients were randomised to receive one of the following treatments for 5 days:
1. GG167 (5mg per inhalation) two oral inhalations twice a day plus placebo two sprays per nostril (0.1ml per spray) twice a day.
2. GG167 (5mg per inhalation) two oral inhalations twice a day plus GG167 (16mg/ml) two sprays per nostril (0.1ml per spray) twice a day.
3. Placebo two oral inhalations twice a day plus placebo two sprays per nostril (0.1ml per spray) twice a day.

GG167 was presented as the formulation of Example 3 using a DISKHALER (trade mark).

The results indicated that GG167 has antiviral activity against influenza virus when administered by inhalation via the mouth alone or in combination with intranasal administration.

### Example 2

### Safety

A double-blind, randomised, placebo-controlled study was conducted in two phases. All treatments were administered to 20 healthy male subjects via a nebuliser.
- Single-Dose Phase:: Eight subjects, aged 18 - 39 years (average 26.5 years, average weight 74.6kg), received single ascending doses of 4mg, 8mg or 16mg GG167 or randomised placebo.
- Multiple-Dose Phase:: Twelve subjects, aged 19 - 45 years (average 28 years, average 75.8kg), received the highest safe and well-tolerated dose as determined in the first phase (i.e. 16mg), or placebo, with up to four administrations per day for 7 days.

GG167, at doses up to 64mg per day, was safe and well tolerated when administered by nebuliser.

### Example 3

### Dry Powder Formulation

- Compound of formula (I) (micronised): 5mg
- Lactose: to 25mg

The formulation is prepared by admixture of the ingredients using conventional pharmaceutical techniques.

## Claims

1. The use of 5-acetamido-2,3,4,5-tetradeoxy-4-guanidino-D-glycero-D-galacto-non-2-enopyranosonic acid for the manufacture of a medicament for administration by inhalation or insufflation through the mouth for the treatment of an animal, including man, suffering from or susceptible to a viral infection.

2. The use as claimed in claim 1 wherein the medicament is 5-acetamido-2,3,4,5-tetradeoxy-4-guanidino-D-glycero-D-galacto-non-2-enopyranosonic acid in the form of a dry powder free from excipients.

3. The use as claimed in claim 1 or 2 wherein the viral infection is an influenza virus infection.

4. The use as claimed in claim 1 wherein the medicament is 5-acetamido-2,3,4,5-tetradeoxy-4-guanidino-D-glycero-D-galacto-non-2-enopyranoasonic acid in the form of a dry powder and further comprising lactose.

5. The use as claimed in claim 4 wherein the medicament contains 5 milligrams (mgs) of 5-acetamido-2,3,4,5-tetradeoxy-4-guanidino-D-glycero-D-galacto-non-2-enopyranosonic acid in the form of a dry powder and further comprising 25 milligrams (mgs) of lactose.

6. The use as claimed in any one of claims 1 to 5 wherein the administration is by inhalation or insufflation through the mouth alone.

## Patentansprüche

1. Verwendung von 5-Acetamido-2,3,4,5-tetradesoxy-4-guanidino-D-glycero-D-galacto-non-2-enopyranosonsäure für die Herstellung eines Medikaments zur Verabreichung durch Inhalation oder Insufflation durch den Mund für die Behandlung eines Tieres, einschließlich eines Menschen, das bzw. der an einer Virusinfektion leidet oder für eine Virusinfektion anfällig ist.

2. Verwendung nach Anspruch 1, wobei das Medikament 5-Acetamido-2,3,4,5-tetradesoxy-4-guanidino-D-glycero-Dgalacto-non-2-enopyranosonsäure in der Form eines von Grundstoffen freien, trockenen Pulvers ist.

3. Verwendung nach Anspruch 1 oder 2, wobei die Virusinfektion eine Grippevirusinfektion ist.

4. Verwendung nach Anspruch 1, wobei das Medikament 5-Acetamido-2,3,4,5-tetradesoxy-4-guanidino-D-glycero-Dgalacto-non-2-enopyranosonsäure in der Form eines trockenen Pulvers ist und des weiteren Lactose aufweist.

5. Verwendung nach Anspruch 4, wobei das Medikament 5 Milligramm (mg) 5-Acetamido-2,3,4,5-tetradesoxy-4-guanidino-D-glycero-D-galacto-non-2-enopyranosonsäure in der Form eines trockenen Pulvers enthält und des weiteren 25 Milligramm (mg) Lactose aufweist.

6. Verwendung nach einem der Ansprüche 1 bis 5, wobei die Verabreichung allein durch Inhalation oder Insufflation durch den Mund erfolgt.

## Revendications

1. Utilisation de l'acide 5-acétoamido-2,3,4,5-tétradésoxy-4-guanidino-D-glycéro-D-galacto-non-2-énopyranosonique pour la fabrication d'un médicament destiné à être administré par inhalation ou insufflation par la bouche pour le traitement d'un animal, y compris l'homme, souffrant d'une infection virale ou sensible à une infection virale.

2. Utilisation selon la revendication 1, dans laquelle le médicament est l'acide 5-acétoamido-2,3,4,5-tétradésoxy-4-guanldino-D-glycéro-D-galacto-non-2-énopyranosonique sous la forme d'une poudre sèche exempte d'excipients.

3. Utilisation selon la revendication 1 ou 2, dans laquelle l'infection virale est une infection virale grippale.

4. Utilisation selon la revendication 1, dans laquelle le médicament est l'acide 5-acétoamido-2,3,4,5-tétradésoxy-4-guanidino-D-glycéro-D-galacto-non-2-énopyranosonique sous la forme d'une poudre sèche comprenant en outre du lactose.

5. Utilisation selon la revendication 4, dans laquelle le médicament contient 5 milligrammes (mg) d'acide 5-acétoamido-2,3,4,5-tétradésoxy-4-guanidino-D-glycéro-D-galacto-non-2-énopyranosonique sous la forme d'une poudre sèche et comprend en outre 25 mg de lactose.

6. Utilisation selon l'une quelconque des revendications 1 à 5, dans laquelle l'administration est seulement effectuée par inhalation ou insufflation par la bouche.
